# EUROPEAN PATENT APPLICATION

(11) **EP 4 056 187 A1**
(43) Date of publication of application: **14.09.2022**
(21) Application number: 21161202.3
(22) Date of filing: 08.03.2021
(51) Int. Cl.: A61K 31/665, A61K 38/12, A61K 9/00, A61P 11/00, A61P 31/04

(54) **ASSOCIATIONS OF FOSFOMYCIN AND COLISTIN FOR USE AGAINST BIOFILM-ASSOCIATED BACTERIAL INFECTIONS**

(71) Applicant: Zambon S.p.A., 20091 Bresso (MI) (IT)
(72) Inventor: COLOMBO, Daniele, 20091 Bresso (MI) (IT); PADOANI, Gloria, 20091 Bresso (MI) (IT); VAILATI, Silvia, 20091 Bresso (MI) (IT); AIEZZA, Noemi, 20091 Bresso (MI) (IT); ANTONELLI, Alberto, 20091 Bresso (MI) (IT); BONCOMPAGNI, Selene, 20091 Bresso (MI) (IT); GIANI, Tommaso, 20091 Bresso (MI) (IT); PALLECCHI, Lucia, 20091 Bresso (MI) (IT); ROSSOLINI, Gian Maria, 20091 Bresso (MI) (IT); MICIELI, Maria, 20091 BRESSO (IT)
(74) Representative: Merli, Silvia

(57) **Abstract**

The present invention relates to a synergistic pharmacological antibiofilm association of colistin and fosfomycin for use in the treatment of a bacterial infection caused by a biofilm-forming pathogen selected from *A. baumannii*, *P. aeruginosa*, *S. maltophilia* and *K. pneumoniae* strains, preferably in the biofilm phase of growth and, in particular infections associated with a respiratory tract disease, such as a chronic respiratory tract disease comprising CF, NCFB and COPD, especially in hospitalized patients.

## Description

### FIELD OF THE INVENTION

The present invention relates to a synergistic pharmacological association of fosfomycin and colistin for use in the treatment of biofilm-associated bacterial infections.

In particular, the present invention relates to a synergistic pharmacological association of fosfomycin and colistin for use in the treatment of a disease related with biofilm-associated bacterial infections caused by one or more Gram-negative biofilm-forming pathogens preferably selected from *A. baumannii, P. aeruginosa, S.maltophilia* and *K. pneumoniae,* especially for use in the treatment of difficult-to-treat respiratory diseases.

### BACKGROUND OF THE INVENTION

Colistin and fosfomycin are "old" antibiotics that have recently regained attention due to the dearth of new compounds to treat infections caused by multidrug-resistant (MDR) pathogens (Karaiskos I. et al., Front Public Health. 2019; 7:151).

Colistin, also termed polymyxin E, a polymyxin antibiotic produced by certain strains of *Bacillus polymyxa,* consists of a cationic cyclic heptapeptide with a tripeptide side chain acylated at the N-terminus by a fatty acid through an α-amide linkage (Reviews of Anti-Infective Agents CID 2005; 40: 1033-41). It disrupts the bacterial membranes, thus resulting in cell death.

Two different forms of colistin are available for clinical use: colistin sulfate which is administered orally for bowel decontamination and topically as a powder for the treatment of bacterial skin and ear infections, and colistimethate sodium (CMS), also called colistin methanesulfate, pentasodium colistimethanesulfate, and colistin sulfonyl methate, for parenteral (intravenous, intramuscular, aerosolized and intrathecal/intraventricular) therapy.

Colistin can be therefore administered as a prodrug in form of colistimethate sodium, which is readily hydrolyzed to form sulfomethylated derivatives, as well as colistin, the active form of the drug.

Colistin has recently gained a crucial role for the treatment of various types of infections, for example pneumonia, bacteremia and urinary tract infections caused by Gram-negative pathogens expressing a MDR phenotype such as, for example, non-fermenting Gram-negative pathogens and carbapenem-resistant enterobacteria.

Gram-negative bacteria are so called because they have a double membrane that does not retain the "Gram" staining and which cannot be penetrated by many antibiotics. Gram-negative bacteria are also more prone to develop Multidrug Resistance, as compared to Gram-positive pathogens.

In patients affected by cystic fibrosis or other chronic respiratory diseases, colistin is commonly used to treat infections caused by *P. aeruginosa* or other MDR non-fermenting Gram-negative pathogens, such as, for example, *S. maltophilia* and *A. baumannii.*

Fosfomycin is an antibiotic which has been used for many decades in the European Union (EU) to treat awide range of infections. For example, it is given by mouth as granules (containing fosfomycin as trometamol salt) or as capsules and powder for oral suspension (fosfomycin calcium), by infusion (drip) into a vein or by injection into the muscle. When given by mouth it is mainly used for treating uncomplicated urinary tract infections caused by bacteria that are vulnerable to fosfomycin's antibacterial effects. In some EU countries it has also been used to prevent infections associated with surgical or diagnostic procedures in the urinary tract. Fosfomycin infusion has been authorised for treating patients of all ages with serious infections such as osteomyelitis (infection of the bone), complicated urinary tract infections, healthcare-associated infections, meningitis and bacterial infections in the blood arising from the other infections. Fosfomycin infusion is reserved for use when other antibiotics cannot be used or are not effective. Fosfomycin for injection into the muscle has been authorised for treating or preventing various infections including infections of the urinary and reproductive systems (EMA/317719/2020 Recommendations to restrict use of fosfomycin antibiotics, 12 June 2020). Fosfomycin acts as a time-dependent inhibitor of the MurA enzyme, which catalyzes the first committed step of the synthesis of peptidoglycans, the main component of the bacterial cell wall.

Fosfomycin-containing medicines are available in most EU countries and are marketed under a variety of names; in particular, fosfomycin trometamol is commercially available under the brand names Monurol^{™} and Monuril^{™} (Zambon S.p.A.).

*A. baumannii, P. aeruginosa, S. maltophilia* and *K. pneumoniae* have emerged as important MDR opportunistic human pathogens responsible for various infections occurring mainly, but not exclusively, in patient suffering from debilitating conditions such as, for example, patients with respiratory diseases including chronic obstructive lung disease (COPD), cystic fibrosis (CF) and non-CF bronchiectasis (NCFB). In addition, these bacteria are increasingly identified in intensive care unit (ICU) as responsible for ventilator-associated pneumonia (VAP), affecting ICU stay and mortality.

Infections caused by *A. baumannii, P. aeruginosa, S. maltophilia* and *K. pneumoniae* are generally chronic and difficult to treat, because of the formidable ability of these bacterial strains to have adapted by existing as adherent populations, that are embedded in a self-produced matrix of Extracellular Polymeric Substances (EPS - biofilm). In fact, biofilm growth confers several advantages to these bacteria and plays an important role in the pathogenesis of the above-defined respiratory diseases and their resistance to antibiotic treatment.

The U.S. Centers for Disease Control estimate that over 65% of nosocomial infections are caused by biofilm-forming pathogens.

To be noted, bacteria inside biofilm are much more resistant to antibiotics than planktonic forms since bacteria that are susceptible to antimicrobial agents in any way can turn resistant after forming biofilm.

Despite a significant amount of time and energy has been devoted to study effective treatments for dealing successfully with bacterial infections caused by emerging biofilm-forming pathogens, there is still substantial lack of good therapeutic options and, hence, it is highly felt in the field the need for more effective treatments of bacterial biofilm-mediated infections.

Biofilms are made of proteins (<1-2%) including enzymes), DNA (<1%), polysaccharides (1-2%) and RNA (<1%), and in addition to these components, water (up to 97%) which is responsible for the flow of nutrients inside biofilm matrix.

Therefore, the two main components are: water channels, which allows nutrients transport, and areas of densely packed cells which may represent up to 4-5% of the EPS matrix.

Biofilm formation, e.g. in *Pseudomonas aeruginosa,* has been recently elucidated (Blasi F. et al. Respiratory Medicine, 2016, 117: 190-197): it is a multi-step process starting with bacterial attachment to a living or non-living surface, then micro-colonies that lead to the formation of a three dimensional structure and start the early stage of biofilm formation, are formed and finally, maturation followed by detachment occurs by rupture of the EPS. During biofilm formation many species of bacteria are able to communicate with one another through a specific mechanism called "quorum sensing", which is a system of intercellular stimulus able to coordinate different gene expression.

It has been ascertained in human pathogens (e.g. *S. aureus:* Becker P. et al., Appl. Env. Microbiology, July 2001, p. 2958-2965 Vol. 67, No. 7. DOI: 10.1128/AEM.67.7.2958-2965.2001) that biofilm formation is due to the activation of bacterial mechanisms and gene transcription which are not common to the planktonic phase.

Bacterial biofilms represent a big threat to the public health because they confer a reduced accessibility to antibiotics and human immune system and are involved in the worsening of a variety of infectious diseases caused by MDR bacteria, which are resistant to more recently developed antibiotics.

In fact, according to National Institutes of Health (NIH) about 65% of all microbial infections, and 80% of all chronic infections are associated with the presence of biofilms.

Therefore is highly felt in the field the need to adopt all means, including new antibiotic combinations suitable for an efficient eradication of biofilm-forming bacteria, which represent a strong criticality worldwide for the emergence of bacterial infections, in particular caused by Gram-negative microorganisms.

### SUMMARY OF THE INVENTION

Accordingly, the present inventors have faced the problem of treating bacterial infections caused by biofilm-forming Gram-negative pathogens, preferably selected from *A. baumannii, P. aeruginosa, S. maltophilia* and *K. pneumoniae,* wherein said infection is particularly difficult to treat due to the presence of biofilms, which are known to impart resistance to antibiotic treatment.

After a long set of tests and experimentations, the present inventors have surprisingly found that the association of colistin and fosfomycin has a synergistic anti-biofilm activity against a number of biofilm-forming *A. baumannii, P. aeruginosa, S. maltophilia* and *K. pneumoniae* clinical isolates.

The anti-biofilm activity of the association of colistin and fosfomycin against biofilm-forming strains of *A. baumannii, P. aeruginosa, S. maltophilia* and *K. pneumoniae,* support the plausibility of the use of said antibiotic association for the treatment of the bacterial colonization of the host, as well as for the treatment of exacerbations elicited by the above mentioned pathogens, especially in hospitalized patients affected by respiratory diseases including, for example, chronic obstructive lung disease (COPD), cystic fibrosis (CF) and non-CF bronchiectasis (NCFB) and ventilator-associated pneumonia (VAP) and exacerbations thereof.

According to a particularly preferred embodiment the association of the present invention is particularly useful for treating infections by Multi-Drug-Resistant (MDR) bacteria.

### DETAILED DESCRIPTION OF THE INVENTION

Represents therefore a first embodiment of the present invention the synergistic pharmacological antibiofilm association of colistin and fosfomycin for use in the treatment of a bacterial infection caused by one or more biofilm-forming pathogen Gram-negative bacteria, preferably selected from the group consisting of: *A. baumannii, P. aeruginosa, S. maltophilia* and *K. pneumoniae,* wherein said pathogens are in the biofilm phase of growth

It is another aspect of the present invention a synergistic pharmacological antibiofilm association of colistin and fosfomycin for use in the treatment of a bacterial infection caused by one or more biofilm-forming pathogen selected from *A. baumannii, P. aeruginosa, S. maltophilia* and *K. pneumoniae,* wherein the infection is associated with a respiratory disease, in particular a chronic respiratory disease.

By pharmacological association of colistin and fosfomycin according to the present invention, is intended the combination of colistin and fosfomycin by either concurrent or combined administration of both active ingredients, which may be packed in a kit comprising suitable doses of colistin and fosfomycin or their physical association in a pharmaceutical composition comprising suitable excipients.

For example in the bacterial infections according to a preferred embodiment of the present invention, i.e. those associated to chronic respiratory diseases, colistin is preferably administered via the inhalatory route, while fosfomycin is preferably administered orally or parenterally, for example i.v..

Accordingly, in a preferred embodiment of the present invention, the kit of parts comprises colistin in powder suitable to be solubilized and administered preferably in either 75 mg or 150 mg dosage at least once, preferably twice a day through a nebulizer and fosfomycin in a dosage suitable to be administered daily orally or parenterally.

Means suitable for solubilizing and diluting colistin and/or fosfomycin are well known by the skilled man and may be sterile physiological solution or, simply, sterile distilled water.

A bacterial infection caused by one or more biofilm-forming pathogen selected from *A. baumannii, P. aeruginosa, S. maltophilia* and *K. pneumoniae* can be detected by standard isolation methods or commercial assays, for example, in patients affected by difficult-to-treat respiratory diseases such as, for example, in patients suffering from COPD, CF, NCFB and VAP and exacerbations thereof especially when these patients are hospitalized patients.

The above aspects are based on the observation of a synergistic interaction between colistin and fosfomycin, which provides a significant antibiofilm activity on biofilm-forming Gram-negative pathogens, preferably selected from the group consisting of: *A. baumannii, P. aeruginosa, S. maltophilia* and *K. pneumoniae* strains.

According to the present invention the term "colistin" comprises colistin and pharmaceutically acceptable salts and/or prodrugs thereof, such as colistin sulfate and colistimethate sodium.

Furthermore, according to the present invention the term "fosfomycin" comprises fosfomycin and its pharmaceutically acceptable salts, such as fosfomycin trometamol (also known as fosfomycin tromethamine), fosfomycin calcium and fosfomycin disodium.

Biofilms and methods suitable for their study have been recently reviewed in Macià et al. (Macià MD et al.. 2014 Oct;20(10):981-90. https://doi.org/10.1111/1469-0691.12651) and Verderosa et al. (Verderosa A. D. et al.,Frontiers in Chemistry, November 28, 2019. https://doi:10.3389/fchem.2019.00824.) because of the growing importance worldwide and in particular in ICUs (Intensive Care Units) of these type of infections. Biofilm-forming pathogens exhibit features, including resistance to antibiotics, which are extremely different from those exhibited in the planktonic growth phase of the same pathogen. These differences explain why many antibiotics, effective on planktonic - rapidly growing - bacteria, are ineffective against the same, wrapped in Extracellular Polymeric Substance (EPS) biofilms, where they also have a reduced growth rate.

As a matter of fact, when a bacterial cell switches to the biofilm phase of growth, it undergoes a phenotypic shift in behavior in which large suites of genes are differentially regulated and expressed (Blasi F. et al. Respiratory Medicine, 2016, 117: 190-197).

The present inventors have observed that combination of colistin and fosfomycin showed a surprisingly remarkable antibiofilm synergism against biofilm-forming Gram-negative pathogens preferably selected from the group consisting of: *A. baumannii, P. aeruginosa, S. maltophilia* and *K. pneumoniae* strains and preferably by using high drug concentrations achievable in the Epithelial Lining Fluid (ELF) after inhalation, which is a preferred route of administration when treating a respiratory disease.

Without being bound to a particular scientific theory, the observed synergistic antibiofilm activity might be due to different mechanisms, such as an increased penetration of both antibiotics in the EPS, or a direct synergic activity on biofilm formation, or a reduced bacterial growth.

According to the present invention the terms "synergistic" and "synergistically" as applied to the effect of colistin and fosfomycin used in association (whether simultaneously or sequentially) refer to a greater antibacterial effect obtained with respect to treatment of the above-identified bacteria by either colistin or fosfomycin alone. In some embodiments, the effect of colistin and fosfomycin used in association (whether simultaneously or sequentially) is greater than the simple addition of the effects of each agent administered alone, i.e. there is an effect which surpasses expectations based on additive effects. Statistical methods, such as those better detailed in the Experimental Part, are available in the field to identify synergy or a simple additive effect.

The activity of colistin and fosfomycin used in association and the synergy observed is particularly important in the treatment of infections by Multi Drug Resistance (MDR) bacteria, against which newly developed antibiotics have shown to be less effective or not effective at all.

Colistin and fosfomycin of the pharmacological association according to the present invention may be administered in either order, separately or concurrently, with overlapping or non-overlapping periods of administration and via the same or different modes of administration.

For example, colistin and fosfomycin can be concurrently administered in a single dosage form or, alternatively, colistin and fosfomycin can be administered in separate dosage forms in either order, concomitantly or sequentially, with overlapping or non-overlapping periods of administration, via the same or different administration route.

The concurrent or separate administration of the pharmacological association of colistin and fosfomycin has the effect of killing pathogens selected from *A*. *baumannii, P. aeruginosa, S. maltophilia* and *K. pneumoniae* grown in biofilms, said effect being unexpectedly and surprisingly greater than what is seen when biofilms are exposed to either colistin or fosfomycin alone, as confirmed in the Experimental Part of the Application.

Accordingly, the effect of this association has been defined "synergic".

The association of colistin and fosfomycin may be administered to the patient in the form of one single or more pharmaceutical compositions.

The pharmaceutical formulations of the present invention comprise colistin and/or fosfomycin together with a carrier suitable for pharmaceutical use, consisting of one or more excipients. For example, the pharmaceutical formulations of the invention may comprise both colistin and fosfomycin together with a carrier suitable for pharmaceutical use, consisting of one or more excipients, i.e. pharmaceutical formulations for concurrent administration of both colistin and fosfomycin; or the pharmaceutical formulations of the invention may comprise fosfomycin together with a carrier suitable for pharmaceutical use, consisting of one or more excipients; or the pharmaceutical formulations of the invention may comprise colistin together with a carrier suitable for pharmaceutical use, consisting of one or more excipients, i.e. separate pharmaceutical formulations for sequential or concomitant administration of colistin and fosfomycin.

According to the present invention, the term "excipient" comprises any inert substance added to a pharmaceutical composition to further facilitate administration of an active ingredient.

According to the present invention, the term "carrier" comprises any substance suitable as a vehicle for delivering colistin and/or fosfomycin to a suitable in vivo or in vitro site.

Pharmaceutically acceptable excipients are those compounds well known to a skilled person in the art that can be used to produce formulations comprising colistin and/or fosfomycin that are suitable to be administered to a subject. Acceptable methods for preparing the pharmaceutical formulations according to the invention are well known to a person skilled in the art.

It is a further object of the present invention a method of treating a bacterial infection caused by one or more biofilm-forming pathogen selected from *A. baumannii, P. aeruginosa, S. maltophilia* and *K. pneumoniae* in a subject in need thereof, which comprises administering to the subject an association of colistin and fosfomycin, wherein the association has a synergistic antibiofilm effect.

It is a further object of the present invention a method of treating a bacterial infection caused by one or more biofilm-forming pathogen selected from *A. baumannii, P. aeruginosa, S. maltophilia* and *K. pneumoniae* in a subject in need thereof, which comprises concurrently administering an association comprising colistin and fosfomycin, wherein the association has a synergistic antibiofilm effect.

It is a still further object of the present invention a method of treating a bacterial infection caused by a biofilm-forming pathogen selected from *A. baumannii, P. aeruginosa, S. maltophilia* and *K. pneumoniae* in a subject in need thereof, which comprises separately administering an association comprising colistin and fosfomycin, wherein the association has a synergistic antibiofilm effect.

According to the present invention, the terms "individual", "subject" and "patient" are used interchangeably to refer to a member of mammalian species, preferably a human, which is afflicted with a particular disease, disorder or condition.

According to the present invention the term "antibiofilm" means the inhibition or suppression of biofilm (formation) or persistence, in biofilm forming bacteria in the biofilm phase of growth.

According to the present invention the term "antibiofilm agent" includes colistin and fosfomycin.

Biofilm formation in the lifecycle of biofilm-forming bacteria can be easily identified by standard methods, such as the checkerboard assay, better detailed in the Experimental Part. (Pollini S. et al. J Antimicrob Chemother 2018; 73: 2388-2395, doi:10.1093/jac/dky185).

According to the present invention, "bacterial infection" refers to any situation in which the presence of a microbial population(s) is harmful to a host mammal. Thus, an individual is "suffering" from a microbial infection when excessive numbers of a microbial population are present in or on an individual's body, or when the effects of the presence of a microbial population(s) is damaging the cells or other tissue of an individual. In particular, "bacterial infection" refers to an infection caused by a strain of bacteria for which the use of a synergistic antibiofilm pharmacological association of colistin and fosfomycin disclosed herein is appropriate.

According to the present invention, the term "nosocomial infection" refers to a hospital-acquired infection (HAI), which is an infection that is acquired in a hospital or other health care facility. To emphasize both hospital and nonhospital settings, it is sometimes instead called a health care-associated infection (HAI or HCAI). Such an infection can be acquired in hospitals, nursing homes, rehabilitation facilities, outpatient clinics, or other clinical settings.

According to the present invention, the terms "treat", "treating" and "treatment" refer to a diminution, a decrease, a limitation, or a mitigation of the degree, intensity, extent of a bacterial infection or its related disease conditions and symptoms caused by one or more biofilm-forming pathogen selected from *A. baumannii, P. aeruginosa, S. maltophilia* and *K. pneumoniae* strains, that is achieved by a reduction, inhibition or suppression of growth, replication, and/or propagation, or death or destruction of said bacteria, on or in the subject.

According to the present invention, the term "pharmacological association" refers to either a fixed combination of colistin and fosfomycin in one unit dosage form, a non-fixed combination or a kit of parts for the combined administration where colistin and fosfomycin, as defined above, may be administered simultaneously, independently at the same time or separately within time intervals that allow the combination partners to show a synergistic effect.

Any suitable route of administration may be used for the compositions of the present invention, including oral, parenteral (subcutaneous, intramuscular, intraperitoneal or intravenous) and inhalation route, preferably the inhalation route.

According to the present invention, the terms "oral" or "orally" refer to the introduction into the body by mouth whereby absorption occurs in one or more of the following areas of the body: the mouth, stomach, small intestine, and the small blood vessels of the oral mucosa.

Non-limiting examples of pharmaceutical formulations according to the present invention for oral administration include, for example, tablets, coated tablets, granulates, pills, capsules, liquids, gels, syrups, suspensions, and the like, for oral ingestion by an individual. Suitable carriers for oral administration are well known in the art.

For parenteral administration, pharmaceutical formulations according to the invention may be formulated for example in aqueous solutions such as in physiologically compatible buffers or physiological salt buffer. Formulations for injection may be presented in unit dosage forms, for example, in ampoules, or in multi-dose containers with, optionally, an added preservative.

For administration by inhalation route, pharmaceutical formulations according to the invention may be formulated in solutions, suspensions and dry powder and delivered by using conventional means, so that optimal quantities of a suitable range of particle sizes are provided to the patient.

According to a particularly preferred embodiment of the invention, the treatment of the above-identified infections is by the inhalation route. In fact, administration by inhalation, allows reaching high drug concentrations in the Epithelial Lining Fluid (ELF) while minimizing systemic toxicity. In particular, colistin is preferably administered by the inhalation route.

Alternatively, the combination of colistin and fosfomycin can be provided by administering one drug by the inhalation route, i.e. colistin, while the other systemically, i.e. orally or parenterally. Particularly preferred is the drug combination in which colistin is administered via the inhalatory route and fosfomycin is administered orally or parenterally.

A further embodiment according to the present invention relates to a method for the therapeutic treatment of a bacterial infection caused by a biofilm-forming Gram-negative pathogen, preferably selected from the group consisting of: *A. baumannii, P. aeruginosa, S. maltophilia* and *K. pneumoniae* in a subject in need thereof, which comprises either administering the combination of colistin and fosfomycin, separately or concurrently, or administering a pharmaceutical composition comprising effective amounts of colistin and fosfomycin together with at least one suitable excipient, wherein said combination has a synergistic antibiofilm effect.

Such a therapeutic treatment may comprise administering an effective amount of colistin, comprised from about 0.075 million units and 12 million units (i.e. from about 6 mg to about 960 mg), preferably between about 0.5 million units and 12 million units (i.e. from about 40 mg to about 960 mg); the effective amount of fosfomycin may vary from 0.1 to 5 g /die, more preferably from 1 mg and 3 mg/die. Pharmaceutical compositions of the present invention may be manufactured in conventional manners, following processes well known in the art.

The amount of colistin and fosfomycin for use according to the present invention may vary depending on the administration route, the selected kind of composition, the individual characteristics of the patient, the duration of the treatment and the nature of concurrent therapies.

For example, the synergistic effective amount of the pharmacological association of colistin and fosfomycin can produce a diminution, a decrease, a limitation, or a mitigation of the degree, intensity, extent of a bacterial infection or its related symptoms caused by one or more biofilm-forming pathogen selected from A. *baumannii, P. aeruginosa, S. maltophilia* and *K. pneumoniae.*

According to one embodiment, the amount of colistin and fosfomycin sufficient to have a synergistic effect on a bacterial infection caused by a biofilm-forming pathogen selected from *A. baumannii, P. aeruginosa, S. maltophilia* and *K. pneumoniae* strains may vary, for example, in view of the physical characteristics of the patient, the severity of the subject's symptoms, the form of the infection, the identity and bacterial load, the formulations and the means used for administering the drug. The specific dose for a given subject is usually set by the judgement of the attending physician.

However, as an example, an effective amount of colistin may be comprised from about 0.075 million units to 12 million units (i.e. from about 6 mg to 960 mg), preferably from about 0.5 million units and 12 million units (i.e. between about 40 mg and 960 mg) to be administered in a single dose or in more repeated doses; the effective amount of fosfomycin may vary from 0.1 to 5 g/die or preferably comprised from 1 to 3 g /die, to be administered in a single dose or in more repeated doses.

Depending on the means of administration a dose may be administered all at once or slowly over a period of time, such as with an i.v. or by inhalation administration.

According to the present invention, the term "dose", "unit dose", "dosage", "effective dose" and related terms refer to physically discrete units that contain a predetermined quantity of active ingredient calculated to produce or induce a desired therapeutic effect. A single dose is thus a predetermined quantity of colistin or fosfomycin that is administered to a patient.

The exact composition, route of administration, and dosage can be chosen by the individual physician in view of the patient's condition, the bacterial load etc. Treatment with the drug combination of the present invention should be continued for as long as required to receive a therapeutic effect according to the invention.

Determination of the synergistic interaction between colistin and fosfomycin was based on the results obtained by the assays described herein below; the *in vitro* antibiofilm synergism between colistin and fosfomycin was determined as better detailed in following examples.

### EXPERIMENTAL PART

### Example 1. Material and methods

### Bacterial strains

Seventeen Gram-negative clinical isolates were investigated, including *A. baumannii* (n=3), *P. aeruginosa* (n=5), *S. maltophilia* (n=3), and *K. pneumoniae* (n=6) (enlisted in Table 1).

The strains were selected for being representative of different genotypes and resistance phenotypes (i.e., Multi-Locus Sequence Typing or MLST-type, colistin and fosfomycin, MDR phenotype). Most of them expressed acquired carbapenem resistance due to carbapenemases production, which currently represent a major clinical challenge for antimicrobial chemotherapy (Bush K, Bradford PA. Clin Microbiol Rev. 2020 Feb 26;33(2):e00047-19. https://doi:10.1128/CMR.00047-19 ).

### Active ingredients

In the experimental part of the present invention, the term "colistin" refers to colistin sulfate (in accordance with the international guidelines for antimicrobial susceptibility testing provided by the Clinical and Laboratory Standards Institute - CLSI and the European Committee on Antimicrobial Susceptibility Testing - EUCAST).

In the experimental part of the present invention, the term "fosfomycin" refers to fosfomycin disodium salt (in accordance with the international guidelines for antimicrobial susceptibility testing provided by the Clinical and Laboratory Standards Institute - CLSI and the European Committee on Antimicrobial Susceptibility Testing - EUCAST).

### Determination of minimum inhibitory concentrations (MICs)

Antimicrobial susceptibility testing was performed according to Clinical and Laboratory Standard Institute guidelines [CLSI M07 2018, CLSI_M100 2020]. In particular, colistin MICs were determined by the broth microdilution method, and fosfomycin MICs by the agar dilution method (with the addition of 25 µg/ml of glucose-6-phosphate, as recommended). All data were obtained in at least two independent experiments, with a third experiment being performed in case of MIC values discrepancies of more than 1 log2 dilution. *E. coli* ATCC 29522 and P. *aeruginosa* ATCC 27853 were used for quality control purposes.

### Biofilm susceptibility testing

Biofilm susceptibility testing was performed using a standardized *in vitro* biofilm model, the Nunc-TSP lid system (Thermo Fisher Scientific, Waltham, MA, USA), as previously described (Harrison JJ, et al. Nat Protoc. 2010;5(7): 1236-1254. https://doi.org/10.1038/nprot.2010.71). Briefly, biofilms were grown in cation-adjusted Mueller Hinton broth (CAMHB) (static conditions, 35°C) for 24 hours, except for *A. baumannii,* which required a 7-days growth in daily refreshed medium (Pollini S et al. J. Antimicrob. Chemother. 2018 Sep 1;73(9):2388-2395. https://doi.org/10.1093/jac/dky185). Preformed biofilms were then exposed to fosfomycin, colistin, and fosfomycin/colistin combinations for 24 hours (static conditions, 35°C) for biofilm susceptibility testing.

Fosfomycin and colistin Minimum Biofilm Eradication Concentrations (MBECs) were determined as previously described (Macià et al, mentioned above), with MBEC being defined as the lowest concentration of antibiotic that prevented visible bacterial growth in the recovery medium.

Biofilm checkerboard assays were performed as previously described (Bonapace CR, et al., Diagn Microbiol Infect Dis. 2002;44(4):363-366. https://doi.org/10.1016/S0732-8893(02)00473-X), using preformed biofilms as the bacterial inoculum. Briefly, after 24 hours of biofilm antibiotic exposure, peg lids were removed and sonicated, and after 24 hours at 35°C were inspected for the presence/absence of bacterial growth in the medium. The synergistic activity of colistin/fosfomycin combinations was evaluated by calculation of the fractional biofilm eradication concentration index (FBECI), where a FBECI value ≤0.5 indicates a synergistic effect (Wang L. et al Frontiers in Microbiology,_2019, 10, 2252, doi: 10.3389/fmicb.2019.02522). Data were obtained in two independent experiments with six replicates per condition per experiment. Median value was used for data analysis.

Finally, all strains were subjected to quantitative biofilm susceptibility testing, using high drugs concentrations achievable by inhalation. Preformed biofilms were exposed to three fosfomycin and three colistin concentrations, alone and in combination. The antibiofilm activity was evaluated by biofilm disruption through sonication, and determination of mean viable cell count per peg (CFU/peg), as previously described (Pollini S. et al. J Antimicrob Chemother 2018; 73: 2388-2395, doi:10.1093/jac/dky185; Ciacci_N. et al., Antibiotics 2019, 8:101; doi:10.3390/antibiotics8030101). Preliminary experiments were performed, in order to determine the optimal range of antibiotic concentrations to be tested for each strain. Data were obtained in at least two independent experiments, with six replicates per condition per experiment. Statistical analysis was performed using the unpaired *t*-test with Welch's correction (GraphPad Prism version 7.0, San Diego, CA, USA), for comparison of the antibiofilm activity of fosfomycin/colistin combinations versus single drugs.

### Example 2. Activity of colistin/fosfomycin combinations against biofilms

MBEC values of fosfomycin and colistin were consistently higher than the respective MICs for all tested strains: i.e. MIC50 = 64 µg/ml vs MBEC50 = >1024 µg/ml, and MIC90 = >1024 µg/ml vs MBEC90 = >1024 µg/ml for fosfomycin; MIC50 = 4 µg/ml vs MBEC50 = 512 µg/ml, and MIC90 = 32 µg/ml vs MBEC90 = >1024 µg/ml for colistin (Table 1).

Biofilm checkerboard assays showed a synergism of fosfomycin/colistin combinations with the majority of tested strains. Furthermore, the synergistic antibiofilm activity of fosfomycin/colistin combinations did not appear to be species-specific or related to fosfomycin and colistin MICs (e.g. *P. aeruginosa* FZ34 and FZ45 strains showed analog fosfomycin and colistin MICs, but discordant response to drug combination exposure) (Table 1). No antagonism was observed.

Quantitative antibiofilm assays (using high drugs concentrations achievable in ELF after inhalation) demonstrated a significant antibiofilm synergism of fosfomycin/colistin combinations against all tested strains, with the exception of two *P. aeruginosa* strains (i.e., FZ34 and FZ45), for which a trend suggesting a synergism was observed, although statistical significance was not achieved. Overall, fosfomycin and colistin MIC values were not predictive of the drug concentrations expressing antibiofilm synergism against a specific strain, emphasizing the complexity of the biofilm response to antibiotic exposure and the differences between the biofilm response and the antibiotic susceptibility.

**Table 1. Results of biofilm checkerboard assays with fosfomycin/colistin combinations:**

| **Strain** | **Species** | **Fosfomycin** | | **Colistin** | | **FBECI** |
|---|---|---|---|---|---|---|
| | | **MIC (µg/ml)** | **MBEC (µg/ml)** | **MIC (µg/ml)** | **MBEC (µg/ml)** | |
| FZ1 | *A. baumannii* | 512 | >1024 | 0.5 | 64 | 0.4 |
| FZ2 | *A. baumannii* | >1024 | >1024 | 1 | >1024 | 0.5 |
| FZ83 | *A. baumannii* | 64 | >1024 | 8 | 128 | 0.3 |
| FZ18 | *P. aeruginosa* | 64 | >1024 | 4 | >1024 | 0.5 |
| FZ34 | *P. aeruginosa* | >1024 | >1024 | 4 | >1024 | 0.5 |
| FZ45 | *P. aeruginosa* | >1024 | >1024 | 4 | >1024 | 1 |
| FZ98 | *P. aeruginosa* | 64 | >1024 | 512 | >1024 | 0.6 |
| FZ139 | *P. aeruginosa* | 8 | >1024 | 4 | >1024 | 0.8 |
| FZ6 | *S. maltophilia* | 64 | >1024 | 4 | >1024 | 0.1 |
| FZ8 | *S. maltophilia* | 128 | >1024 | 4 | >1024 | 0.3 |
| FZ85 | *S. maltophilia* | 32 | >1024 | 16 | >1024 | 0.8 |
| FZ80 | *K. pneumoniae* | 128 | >1024 | 0.5 | 64 | 0.6 |
| FZ103 | *K. pneumoniae* | 32 | >1024 | 8 | 64 | 0.1 |
| FZ105 | *K. pneumoniae* | 16 | >1024 | 128 | 256 | 0.1 |
| FZ106 | *K. pneumoniae* | 64 | >1024 | 32 | 256 | 0.2 |
| FZ108 | *K. pneumoniae* | 128 | >1024 | 32 | >1024 | 0.1 |
| FZ141 | *K. pneumoniae* | ≤0.25 | 4 | 2 | 8 | 0.4 |

| | | | | | | |
|---|---|---|---|---|---|---|
| Abbreviations: MIC, minimum inhibitory concentration; MBEC, minimum biofilm eradication concentration; FBECI, fractional biofilm eradication concentration index. FBECI values were interpreted as follows: FBECI ≤0.5, synergy; FBECI >0.5 - 4.0, no interaction; FBECI >4.0, antagonism. FBECI values indicating synergy are shown in bold. | | | | | | |

## Claims

1. A synergistic pharmacological combination of colistin and fosfomycin for use in the treatment of a bacterial infection caused by one or more Gram negative biofilm-forming pathogen preferably selected from the group consisting of: *A. baumannii, P. aeruginosa, S. maltophilia* and *K. pneumoniae,* wherein said pathogens are in the biofilm phase of growth.

2. The combination for use according to claim 1 wherein the infection is associated with a respiratory disease preferaby a chronic respiratory disease.

3. The combination for use according to claim 2 wherein said chronic respiratory disease is selected from the group consisting of: Chronic Obstructive Polmunary Disease (COPD), Cystic fibrosis (CF), Non Cystic Fibrosis (NCFB), Ventilator-Associated Pneumonia (VAP) and exacerbations thereof.

4. The combination for use according to claim 1 wherein said bacterial infections are present in hospitalized or Intensive Care Unit patients.

5. The combination for use according to any one of claims 1-4 wherein said pathogens are Multi Drug Resistant bacteria.

6. The combination for use according to any one of claims 1-5 wherein colistin and fosfomycin are administered in either order, separately or concurrently, with overlapping or non-overlapping periods of administration, via the same or different administration route.

7. The combination for use according to claim 6 wherein said administration route is the inhalatory route.

8. The combination for use according to claim 6 wherein colistin is administered by the inhalatory route and fosfomycin is administered by the oral or parenteral route.

9. A kit of parts comprising a vial of colistin and a vial of fosfomycin optionally in combination with dilution means, for the combined administration of the synergic combination of colistin and fosfomycin.

10. The kit of parts according to claim 9 wherein colistin is diluted for administration via the inhalation route and fosfomycin is diluted for administration via the oral or parenteral route.

11. Use of the synergistic pharmacological association of colistin and fosfomycin for the preparation of an antibiofilm medicament for the treatment of Gram-negative infections by a pathogen preferably selected from the group consisting of: *A. baumannii, P. aeruginosa, S. maltophilia* and *K. pneumoniae,* wherein said pathogen is in the biofilm phase of growth.
